# FASCICULE DE BREVET EUROPEEN

(11) **EP 0 550 361 B1**
(45) Date de publication et mention de la délivrance du brevet: **16.10.1996**
(21) Numéro de dépôt: 92420488.6
(22) Date de dépôt: 31.12.1992
(51) Int. Cl.: C07C 219/06, C07C 231/14, C07C 213/06, C11D 1/62, D06M 13/463, B01F 17/00

(54) **Tensioactifs à base de composés d'ammonium quaternaire, procédés de préparation, bases et compositions assouplissantes dérivées**
Quaternär-Ammonium Tenside, Verfahren zu ihrer Herstellung, Basen und ihre ableitenden Weichmacher
Surface active quaternary ammonium compounds, processes for their preparation and softening compositions derived from them

(30) Priorité: 31.12.1991 FR 9116479; 12.03.1992 FR 9203199; 17.07.1992 FR 9209067
(43) Date de publication de la demande: 07.07.1993
(73) Titulaire: STEPAN EUROPE, F-38340 Voreppe (FR)
(72) Inventeur: Contet, Jean-Pierre, F-38430 St Jean de Moirans (FR); Courdavault Duprat, Stéphane, F-38240 Meylan (FR); Godefroy, Lionel, F-38340 Moirans (FR); Nivollet, Paul, F-38340 Voreppe (FR); Ray, Didier, F-38120 Saint Egreve (FR); Storet, Yvan, F-38113 Veurey-Voroize (FR); Vindret, Jean-François, F-38380 Saint Laurent du Pont (FR)
(74) Mandataire: Guerre, Dominique

(56) Documents cités:
- EP-A- 0 075 168
- EP-A- 0 131 865
- EP-A- 0 284 036
- WO-A-91/17974
- DE-A- 2 641 286
- US-A- 2 589 674
- US-A- 2 935 474
- US-A- 3 915 867

## Description

Le domaine de la présente invention est celui des compositions adoucissantes ou assouplissantes, notamment pour textiles, comprenant à titre d'agent actif au moins un tensioactif cationique du type ammonium quaternaire.

Selon le document EP-0 284 036, il est proposé un produit, sous forme de sel d'ammonium quaternaire, résultant de la condensation entre :
- d'une part, une fraction d'acides gras, saturés ou insaturés, linéaires ou ramifiés, sous forme d'esters de glycérol et de préférence triesters de glycérides, lesdits acides gras présentant chacun une chaîne hydrocarbonée dont le nombre d'atomes de carbone est compris entre 5 et 23,
- et d'autre part, au moins une amine tertiaire fonctionnalisée, dont les chaînes alkyle comportent ensemble au moins quatre atomes de carbone et répondent à la formule (III) suivante : 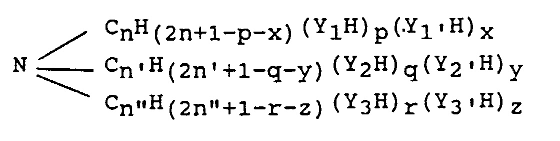 dans laquelle:
- 4 < n + n'+ n" < 11,
- 1 < p + q + r + x + y + z < 3,
   avec p + x = 1, q + y et r + z égaux à 0 ou 1
- Y₁, Y_{1'}, Y₂, Y_{2'}, Y₃, Y_{3'} sont des groupes fonctionnels oxy.

Le rapport molaire fraction d'acides gras/amine tertiaire est préférentiellement égal à 1 pour obtenir un produit de condensation monoestérifié, et égal à 2 pour obtenir un produit de condensation diestérifié. C'est sous la forme diester, de préférence, que le produit de condensation trouve une application en tant qu'assouplissant et adoucissant pour textile.

La demande de brevet internationale WO-91/17974 concerne un composé d'ammonium quaternaire obtenu par réaction d'acides gras insaturés ayant de 16 à 22 atomes de carbone et ayant au moins 40 % de doubles liaisons insaturées de configuration trans, ou dérivés, avec une amine répondant à la formule A : 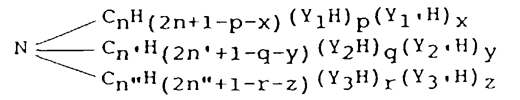 dans laquelle:
- 5 ≤ n + n'+ n" ≤ 30
- 2 ≤ p + q + r + x + y + z ≤ 3
- Y₁, Y_{1'}, Y₂, Y_{2'}, Y₃, Y_{3'} sont des groupes fonctionnels oxy.

Le rapport molaire fraction d'acide gras/amine tertiaire est compris entre 1,2 et 2,5.

La demande de brevet DE-A-2 641286 décrit un composé d'ammonium quaternaire répondant à la formule C suivante : 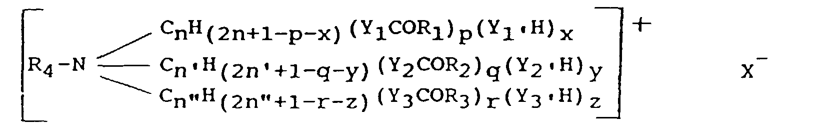 dans laquelle:
- n + n'+ n" = 7
- p + q + r + x + y + z = 3
- 1 ≤ p + q + r ≤ 3
   avec p + x = q + y = 1 et z = 0 et r = 1
- R₁ et R₂ sont H ou R₃
- R₃ est une chaîne carbonée saturée ou insaturée ayant au moins 11 atomes de carbone
- R₄ est une chaîne ou cycle carboné substitué ou non ayant de 1 à 22 atomes de carbone
- Y₁, Y_{1'}, Y₂, Y_{2'} sont des groupes fonctionnels oxy.
- Y₃ est un groupe fonctionnel aminé
- X est un contre-ion.

Selon que l'on veut obtenir essentiellement l'amide, l'amide-monoester ou l'amide-diester, le rapport molaire fraction acide gras/amine tertiaire varie de 1 à 3.

Le produit de condensation obtenu est utilisable en concentrations relativement importantes, dans des assouplissants textiles concentrés. Une telle utilisation se heurte cependant à deux inconvénients majeurs, celui du manque de fluidité et celui du manque de stabilité. Et la préparation de compositions à viscosité et stabilité données avec de tels produits implique la nécessité d'abaisser la concentration de ces derniers.

La présente invention a pour objet un produit tensioactif pour assouplissant textile, de la famille chimique identifiée ci-dessus, qui, même en l'absence d'additifs complexes, présente à la fois une bonne stabilité, et une bonne fluidité ou "coulabilité", sous des concentrations relativement importantes.

Selon la présente invention, on a trouvé que de tels composés tensioactifs pouvaient être obtenus par le choix d'un rapport molaire fraction d'acide gras/amine tertiaire compris entre 1,85 et 1,40, avec des amines tertiaires dont les chaînes alkyles comportent jusqu'à 12 atomes de carbone et une à quatre fonctions choisies dans le groupe comprenant les fonctions hydroxyle et amine.

On a en effet constaté que pour un rapport molaire fraction acides gras/amine tertiaire supérieur à 1,85, les formulations concentrées sont moins fluides et surtout moins stables et que pour le même rapport inférieur à 1,4, le pouvoir conditionnant et lubrifiant est moins bon.

Les acides gras sont sous forme libre ou dérivés; dans ce dernier cas les dérivés sont choisis dans le groupe comprenant les esters, anhydrides et chlorures d'acide ou bien sous forme d'esters de glycérol et de préférence triesters d'acides gras et de glycérol.

Préférentiellement, la fraction d'acides gras comporte au moins 50 % en moles d'acides linéaires en C₁₆ à C₁₈, saturés ou insaturés. Ceci permet de préserver un pouvoir assouplissant acceptable.

Lorsque la fraction d'acides gras C₁₆/C₁₈ comporte majoritairement des acides linéaires en C₁₈, le rapport molaire fraction d'acides gras/amine tertiaire est compris préférentiellement entre 1,82 et 1,60. Lorsque la fraction d'acides gras C₁₆/C₁₈ comporte majoritairement des acides linéaires en C₁₆, le rapport molaire fraction d'acides gras/amine tertiaire est compris préférentiellement entre 1,85 et 1,66.

Lorsque la fraction d'acides gras est au moins partiellement insaturée, les deux modes suivants d'exécution de la présente invention sont préférés :
- lorsque la fraction d'acides gras C₁₆/C₁₈ comporte majoritairement des acides linéaires en C₁₈, cette fraction présente une insaturation globale, exprimée par un indice d'iode (selon la norme française NF ISO 3961 de février 1990 de l'AFNOR) compris entre 10 et 33, et notamment entre 15 et 33
- lorsque la fraction d'acides gras C₁₆/C₁₈ comporte majoritairement des acides linéaires en C₁₆, cette fraction présente une insaturation globale, exprimée par un indice d'iode compris entre 10 et 43, et notamment entre 15 et 35.

Selon la présente invention, il a en effet été mis en évidence qu'une insaturation trop faible augmentait la viscosité et l'instabilité de formulations concentrées, et qu'une insaturation trop forte diminuait le pouvoir assouplissant et augmentait l'odeur résiduelle au stockage.

Selon un autre mode d'exécution de l'invention, un composé tensioactif résulte de la condensation d'une fraction d'acides gras avec un mélange d'une amine tertiaire polyhydroxylée et d'une amine tertiaire substituée par une fonction hydroxyle ou amine, et ce dans un rapport molaire de la première par rapport à la seconde au moins égal à 2.

Dans ce dernier cas, lorsque la fraction d'acides gras C₁₆/C₁₈ comporte majoritairement des acides linéaires en C₁₆, l'insaturation globale de cette dernière est inférieure à 45, et notamment inférieure à 35, toujours exprimée par l'indice d'iode de ladite fraction ; lorsque cette même fraction d'acides gras C₁₆/C₁₈ comporte majoritairement des acides linéaires en C₁₈, celle-ci présente une insaturation globale, exprimée par un indice d'iode inférieur à 33.

Une molécule élémentaire d'un tensioactif selon l'invention; répond à la formule chimique générique précédente (I), 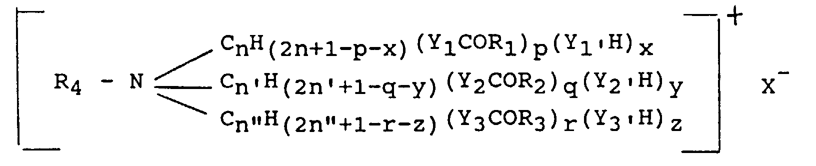 dans laquelle :
- n, n' et n'' sont des nombres entiers non nuls dont la moyenne de la somme est au moins égale à 4, rapportée à 1 mole de tensioactif,
- p, q, r, x, y et z sont des nombres entiers ou zéro,
- Y₁, Y_{1'}, Y₂, Y_{2'}, Y₃, Y_{3'} sont des groupes fonctionnels,
- R₁, R₂, R₃ sont respectivement une longue chaîne hydrocarbonée alkyle ou alkényle, ayant 5 à 23 atomes de carbone,
- R₄ est une courte chaîne alkyle ayant de 1 à 5 atomes de carbone, et
- X est un contre-ion,
et se caractérise par le choix du paramètre chimique suivant :
- rapportée à une mole, la moyenne de la somme de p, q et r est comprise statistiquement entre 1,85 et 1,40, la moyenne de ladite somme de n, n' et n'' est statistiquement au plus égale à 12, rapportée à une mole et, les groupes fonctionnels Y₁, Y_{1'}, Y₂, Y_{2'}, Y₃, Y_{3'} sont choisis chacun parmi les groupes oxy, N et NH, et étant ensemble, en nombre compris entre 1 et 4.

Un tensioactif préféré de l'invention est celui qui consiste en un mélange d'au moins deux composés répondant à la formule (I) dans laquelle :
- Y₁, Y_{1'}, Y₂, Y_{2'}, Y₃, Y_{3'} sont des groupes oxy, et
- (p + q + r) est comprise entre 1,82 et 1,60 quand les longues chaînes R_{1,} R₂, R₃ sont majoritairement des chaînes en C₁₇, ou est comprise entre 1,85 et 1,66 quand les longues chaînes R_{1,} R₂, R₃ sont majoritairement des chaînes en C₁₅.

Un autre tensioactif préféré de l'invention est celui qui consiste en un mélange d'au moins deux composés répondant à la formule (I) dans laquelle :
- Y₁, Y_{1'}, Y₂, Y_{2'}, Y₃, Y_{3'} sont des groupes oxy, et
- l'indice d'iode rapporté aux acides gras correspondant à R_{1,} R₂, R₃ est compris entre 15 et 35 quand ils sont majoritairement en chaînes à C₁₅, et est compris entre 15 et 33 quand ils sont majoritairement en chaînes à C₁₇.

En plus de sa stabilité, le tensioactif de l'invention est particulièrement avantageux en ce que son utilisation, même en concentrations élevées n'engendre pas de rejets toxiques dans l'environnement, car le composé de l'invention qui le constitue'est biodégradable.

Par biodégradable, on entend aussi bien la biodégradabilité primaire, c'est-à-dire la perte de pouvoir tensioactif qui doit être d'au moins 80 % en test statique, dit "bottle test", notamment selon la norme française expérimentale de l'AFNOR, Pr T73280, que la biodégradabilité totale, par suivi de la perte d'au moins 80 % du carbone organique dissous, par exemple par un des tests dynamiques de confirmation de l'OCDE, notamment décrit dans JOCE (Journal Officiel des Communautés Economiques Européennes) L 133/106 du 30/05/1988, ou par tout autre test équivalent.

Pour son utilisation, sous forme d'une base assouplissante, un tensioactif selon l'invention peut être dissous dans un ou des solvants choisis parmi les alcools inférieurs et notamment méthanol, éthanol, isopropanol, parmi les glycols et notamment éthylène-glycol, propylène-glycol, diéthylène-glycol, butyl-, propyl-, éthyl-, méthyl-éther de diméthylène glycol, de diéthylène glycol, de dipropylène glycol, hexylène-glycol, parmi l'eau et la glycérine, mais aussi parmi les composés répondant à la formule (II) suivante :

(R₅ - E)ₘ- Cⱼ H_{2(j+1)-m-2d+i} Oₖ₊ᵢ (II)

dans laquelle :
- R₅ est une longue chaîne hydrocarbonée saturée ou insaturée, ayant de 5 à 23 atomes de carbone
- E est une fonction 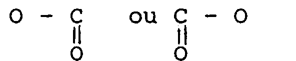
- m est un nombre entier compris entre 1 et 8
- j est un nombre entier compris entre 1 et 36
- k est un nombre entier ou zéro
- i est un nombre entier ou zéro
- d est égal à 0, 1 ou 2.

Dans la formule II ci-dessus :
- k représente le nombre de fonctions éther du composé
- i représente le nombre de groupements hydroxyle libre du composé
- et d représente le nombre d'insaturations du composé, non comprises celles de R₅.

Le composé II préférentiel ne comprend pas de groupements hydroxyle libre, c'est-à-dire i est égal à zéro.

Avantageusement, le composé répondant à la formule II est choisi parmi ceux pour lesquels :
- m est égal à 1, j est compris entre 1 et 18 et de préférence entre 1 et 4, et k, i et d sont égaux à 0, c'est-à-dire notamment parmi les esters méthyliques, éthyliques, propyliques, isopropyliques et butyliques ; pour des valeurs de m et j égales à 1, R₅ est une chaîne hydrocarbonée ayant de préférence 7 à 17 atomes de carbone
- m est égal à 2, j est compris entre 2 et 12 et est de préférence égal à 2 ou 3, et k, i et d sont égaux à 0, c'est-à-dire notamment parmi les diesters d'éthylène glycol et de propylène glycol
- m est égal à 2, j est compris entre 4 et 12, k est égal à 1, i et d sont égaux à 0 et R₅ est une chaîne hydrocarbonée ayant 7 ou 9 atomes de carbone, tels que l'ester de diéthylèneglycol de l'acide caprique ou caprylique
- m est égal à 3, j est égal à 3 ou 4, k, i et d sont égaux à 0, et R₅ est une chaîne hydrocarbonée ayant 7 ou 9 atomes de carbone, tels que le triester de glycérol de l'acide caprique ou caprylique
- m est égal à 3, j est égal à 6 et k, i et d sont égaux à 0, tels que le triester de triméthylol-propane
- m est égal à 4, j est égal à 5, et k, i et d sont égaux à 0, tels que le tétraester de pentaérythritol.

L'utilisation d'un solvant comprenant au moins un composé répondant à la formule (II), présente les avantages suivants :
- en plus de son pouvoir dissolvant, il apporte un effet lubrifiant et un effet conditionnant complémentaires de ceux apportés par le tensioactif répondant à la formule (I),
- les composés II ont un point éclair élevé permettant d'augmenter celui de la base tensioactive résultante, rendant ainsi son utilisation plus sûre, même à température élevée,
- les composés II sont biodégradables.

Une base assouplissante de l'invention comprend avantageusement 60 à 99% en poids d'au moins un composé répondant à la formule (I) et 40 à 1% en poids d'au moins un composé de la formule (II) à titre de solvant.

Quand la base assouplissante comprend plus d'un composé tensioactif, elle peut comprendre un mélange de composés tensioactifs différents ou un mélange de composés tensioactifs homologues, indépendamment de la nature des acides gras, chacun des composés tensioactifs répondant à la formule (I).

Un tel mélange peut être obtenu, soit en préparant séparément les deux tensioactifs distincts, soit préférentiellement, par une seule et même réaction de condensation des fractions d'acides gras d'une part, et du mélange des amines tertiaires différentes, d'autre part.

Un autre objet de l'invention est une composition assouplissante comprenant une base assouplissante telle que décrite précédemment.

Une composition assouplissante de l'invention, incorporée dans la formule d'un assouplissant textile par exemple est stable aussi bien chimiquement, c'est-à-dire ne présentant pas de décomposition importante quand elle est stockée à 20°C, que physiquement, c'est-à-dire ne présentant pas de séparation marquée de phases ou de modifications importantes de la viscosité ou de l'odeur.

Une composition assouplissante selon l'invention peut en outre comprendre les additifs courants selon sa destination, tels que parfums, colorants, azurants, protecteurs...

Une fois incorporées dans des assouplissants de fibres textiles, les compositions de l'invention présentent des viscosités appropriées pour une utilisation en machine à laver ménagère courante, c'est-à-dire inférieures à 700 mPa.s (mesurées sur un viscosimètre Contraves TV mobile 3, ou sur tout autre appareil donnant un taux de cisaillement équivalent).

Toute composition assouplissante selon l'invention permet dans le produit final d'atteindre une concentration pondérale en tensioactifs comprise entre 3 et 30 % en poids sans qu'aucun problème de présentation et d'usage n'apparaisse.

Lorsque les tensioactifs qu'elle comprend sont obtenus par condensation en une seule réaction, entre une fraction d'acides gras sous forme de triesters de glycérol et au moins une amine, puis par quaternisation, le solde de ladite composition comprend des produits secondaires susceptibles d'être obtenus par la réaction de condensation, tels que des monoglycérides, des diglycérides, du glycérol, et/ou des produits secondaires susceptibles d'être obtenus par la réaction de quaternisation, dont ledit produit non quaternisé.

Enfin, l'invention a pour dernier objet un procédé pour préparer un tensioactif.

Selon ce procédé, on fait réagir les acides gras ou leurs dérivés esters, anhydrides et chlorures, lesdits acides gras étant sous forme libre ou sous forme d'esters de glycérol et de préférence triesters de glycérol, et lesdits acides gras répondant respectivement aux formules R₁COOH, R₂COOH, R₃COOH, dans lesquelles R₁, R₂, R₃ sont respectivement une longue chaîne hydrocarbonée alkyle ou alkényle, linéaire ou ramifiée, ayant de 5 à 23 atomes de carbone, avec au moins une amine tertiaire répondant à la formule (III) suivante : 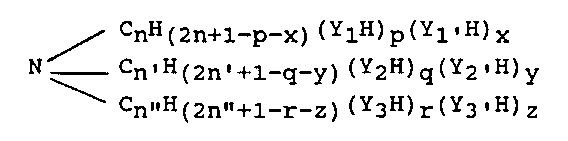 dans laquelle:
- n, n', n'' sont des nombres entiers non nuls,
- p, q, r, x, y et z sont des nombres entiers ou zéro,
- rapportée à une mole, la moyenne de la somme de n, n' et n" est au moins égale à 4,
- Y₁, Y_{1'}, Y₂, Y_{2'}, Y₃, Y_{3'} sont des groupes fonctionnels,
puis à faire réagir le produit de condensation ainsi obtenu avec un agent alkylant, en présence ou non d'un solvant.

Si les acides gras ou dérivés sont sous forme libre, le procédé de l'invention se caractérise par le rapport de la concentration molaire des acides gras à celle des amines tertiaires, qui est compris entre 1,85 et 1,40, et dans la formule (III) de chaque amine tertiaire,
- la somme de n, n', n" est comprise entre 4 et 12,
- la somme de p, q, r, x, y et z est en moyenne statistique comprise entre 1 et 4,
- Y₁, Y_{1'}, Y₂, Y_{2'}, Y₃, Y_{3'} sont choisis chacun parmi les groupes oxy, N et NH.

Les acides gras de départ sont de préférence choisis parmi les acides gras d'origine animale tels que le suif, et les acides gras d'origine végétale tels que ceux de palme, colza, soja et tournesol.

Les amines tertiaires de départ, quand la somme de p, q, r, x, y et z est supérieure à 1, sont de préférence choisies parmi la triéthanolamine, la méthyldiéthanolamine, l'éthyldiéthanolamine, le diméthylamino-N-2,3-propanediol, le diéthylamino-N-2,3-propanediol, le méthylamino-N,N-bis-2,3-propanediol et l'éthylamino-N,N-bis-2,3-propanediol, et quand la somme de p, q, r, x, y et z est égale à 1, elles sont de préférence choisies parmi la diméthylaminopropylamine et la diméthylaminoéthanolamine, et la diéthylaminoéthanolamine.

Selon une mise en oeuvre préférentielle du procédé, les amines tertiaires sont en mélange et le rapport molaire des amines tertiaires pour lesquelles la somme de p, q, r, x, y et z est strictement supérieure à 1, aux amines tertiaires pour lesquelles la somme de p, q, r, x, y et z est égale à 1, est au moins égal à 2.

L'agent alkylant est préférentiellement choisi parmi les halogénures, les sulfates, les phosphates et carbonates d'alkyle.

Si les acides gras sont sous forme de triesters de glycérol, le procédé de l'invention se caractérise en ce qu'on fait réagir partiellement l'amine tertaire, selon une transestérification et/ou transestéramidification, avec le glycéride, et :
- rapportée à une mole, la moyenne de la somme de n, n' et n'' est comprise statistiquement entre 4 et 12,
- rapportée à une mole, la moyenne de la somme de p, q, r, x, y et z étant comprise statistiquement entre 1 et 4,
- Y₁, Y_{1'}, Y₂, Y_{2'}, Y₃, Y_{3'} étant choisis chacun parmi les groupes oxy, N et NH.

De manière préférentielle, le rapport molaire du triester de glycérol mis en réaction, à celui de l'amine tertiaire est compris entre 1/3 et 1.

De cette manière, après quaternisation, on obtient une composition assouplissante qui s'est révélée directement utilisable, et donc économique à préparer, puisqu'en particulier les produits secondaires précités qu'elle peut contenir sont en grande partie actifs vis-à-vis des applications considérées, et apportent des effets complémentaires, tels qu'un effet lubrifiant, adoucissant, épaississant, hydratant, tout en jouant pour partie le rôle d'un solvant.

Le procédé selon l'invention se prête à une industrialisation dans des conditions économiques, d'une part parce qu'il utilise une matière première peu élaborée et abondante, en l'occurrence au moins un triester de glycérol, ou triester de glycérol tel que huile de palme, et d'autre part parce qu'au moins une étape réactionnelle est limitée, et donc plus courte.

Pour obtenir une composition assouplissante selon l'invention, selon ce procédé, le rapport molaire du triester de glycérol à l'amine tertiaire est de préférence compris entre 1/3 et 1.

Le procédé de transestérification et/ou transesteramidification comporte les caractéristiques techniques complémentaires suivantes :
- le solde de la composition ou du mélange tensioactif comprend des produits secondaires de la réaction partielle, c'est-à-dire non complète de quaternisation du produit de condensation, à savoir des alkyl-ester (et/ou amido)-amines non quaternisées; certains de ces produits se sont révélés en effet actifs, et pour certains d'entre eux tensio-actifs, de telle sorte qu'il peut être intéressant de limiter ou contrôler la réaction de quaternisation,
- l'agent alkylant est préférentiellement choisi parmi les halogénures d'alkyle ou d'alkylbenzyle, tels que le chlorure de méthyle ou le chlorure de benzyle, les sulfates d'alkyle, du type polyalkylestersulfates, tels que les diméthyl ou diéthylsulfates, les phosphates d'alkyle, et les carbonates d'alkyle, tels que le diméthylcarbonate,
- les acides gras du triester de glycérol comprennent préférentiellement au moins 50 % en moles d'acides linéaires en C₁₆ à C₁₈,
- l'étape de transestérification et/ou transesteramidification s'effectue ou non en présence de catalyseurs usuels, notamment acides ou basiques,
- la composition ou mélange tensioactif comprend ou non des antioxydants, utilisés lors de l'étape de transestérification et/ou transesteramidification, notamment du butylhydroxytoluène ou du butylhydroxyanisol,
- la composition ou mélange tensioactif comprend ou non un agent décolorant, réducteur ou oxydant,
- la composition ou mélange tensioactif comprend ou non différents agents modificateurs ou protecteurs, tels que acides citriques, acides tartriques, hydrures simples ou mixtes,
- la composition ou mélange tensioactif comprend ou non un agent complexant,
- un solvant complémentaire, notamment du type alcool, glycol ou ester, peut être ajouté à la composition, pour en modifier la présentation et certaines caractéristiques,
- s'agissant de l'amine tertiaire de formule (II), utilisée dans le processus réactionnel selon l'invention, la somme des indices p, q, r, x, y et z de l'amine tertiaire est au plus égale à 4,
- l'amine tertiaire de départ est préférentiellement polyfonctionnelle, c'est-à-dire comporte plusieurs fonctions hydroxyle ou amine primaire ou secondaire, et est choisie notamment parmi le N,N-diéthylamino-2,3-propane diol, le N,N-diméthylamino-2,3-propane diol, la N-éthyldiéthanolamine, et la triéthanolamine,
- l'amine tertiaire est aussi éventuellement monofonctionnelle et peut être aussi associée à une amine polyfonctionnelle, c'est-à-dire comporte une seule fonction hydroxyle ou amine primaire ou secondaire, et est notamment choisie dans le groupe comprenant :
   la N,N-diméthylaminopropylamine
   la N,N-diéthylaminopropylamine.
   la N,N-diméthylaminoéthanolamine
   la N,N-diéthylaminoéthanolamine
   la N,N-diméthylaminoisopropanolamine
   la N,N-diéthylaminoisopropanolamine,
   dans ce cas, avantageusement le rapport amine tertiaire polyfonctionnelle/amine tertiaire monofonctionnelle est compris entre 2 et 200
- les acides gras retenus sous forme de triester de glycérol sont notamment des huiles ou graisses d'origine naturelle, raffinées ou non, partiellement, totalement ou pas du tout hydrogénées, telles que le suif, le saindoux, l'huile de coprah, l'huile de palme, l'huile de soja, l'huile de poisson, l'huile de colza, et l'huile de tournesol.

Les différents objets de l'invention sont à présent abordés en détail à l'appui des Exemples 1 à 15 suivants, illustrant pour les Exemples 1 à 8, la préparation de tensioactifs selon l'invention, les Exemples 1 à 5 correspondant à une préparation à partir d'acides gras sous forme libre ou dérivés, les Exemples 6 à 8 correspondant à une préparation à partir d'acides gras sous forme de triesters de glycérol, pour les Exemples 9 à 12, on prépare des bases tensioactives avec un composé tensioactif selon l'invention, dissous dans un solvant dont au moins un composé répond à la formule (II), et pour les Exemples 13 à 15, on prépare des compositions assouplissantes selon l'invention.

Le procédé de l'invention n'est bien sûr pas limité aux conditions opératoires utilisées dans ces exemples, concernant en particulier les températures de réaction qui peuvent être inférieures à 100°C ou supérieures à 200°C, et le choix des catalyseurs et additifs, notamment protecteurs ou décolorants. Le choix de ces derniers peut être effectué, parmi les acides forts, les bases fortes, les hydrures alcalins, les alcoolats alcalins, ou d'autres métaux tels que le titane, le zinc, (titanate...), les chlorites ou hypochlorites, phosphites, hypophosphites, sulfites, hyposulfites, d'alcalins ou d'autres métaux ou les formes acides lorsqu'elles existent, le butylhydroxytoluène, le butylhydroxyanisol, les citrates, tartrates, lactates, gluconates, lactobionates, phosphonates ou autres complexants, sans que cette liste soit limitative.

Dans les Exemples 2, 3, 4, 5 et 10, le taux d'insaturation global de l'ensemble des chaînes R₁, R₂, R₃ est évalué par l'indice d'iode, qui est mesuré selon la norme française AFNOR NF-ISO 3961 (février 1990) et exprimé en gramme d'iode pour 100 grammes de composé testé.

### EXEMPLE 1

Préparation du tensioactif appelé "quat n°1" constitué par un mélange de composés répondant à la formule I dans laquelle :
- n + n' + n" = 6
- p + q + r + x + y + z = 3
- Y₁, Y_{1'}, Y₂, Y_{2'}, Y₃, Y_{3'} sont des groupes oxy
- R₄ représente CH₃
- X⁻ représente CH₃SO₄⁻

### Estérification :

540 grammes d'acides gras de palme sont chargés en atmosphère inerte dans un réacteur en acier inox, 172 g de triéthanolamine sont ajoutés sous agitation. Le mélange est chauffé au moins 12 heures à 140°C sous vide partiel pour éliminer l'eau de réaction. L'avancement de la réaction est suivi par un dosage acide/base qui détermine l'acidité résiduelle pour obtenir une estérification d'au moins 90 à 95 % des acides gras.

676 g d'un produit jaune liquide à chaud appelé "esteramine 1" sont récupérés, constitué essentiellement d'un mélange d'amine non estérifiée, mono, di et tri estérifiée.

### Quaternisation :

A 676 g d'"esteramine 1" sont ajoutés sous agitation 140 g de diméthylsulfate à une température de 60-80°C. Après une heure de digestion, l'absence quasi totale d'amine résiduelle est vérifiée par dosage acide/base. On obtient 816 g d'esteramine 1 quaternisée. Le produit est ensuite dilué à 90 % par ajout de 11 % environ en poids d'isopropanol ; on obtient 906 g de tensioactif appelé "quat n°1".

### EXEMPLE 2

Préparation du tensioactif appelé "quat n°2" constitué par un mélange de composés répondant à la formule I dans laquelle :
- n + n' + n" = 6
- p + q + r + x + y + z = 3
- Y₁, Y_{1'}, Y₂, Y_{2'}, Y₃, Y_{3'} sont des groupes oxy
- R₄ représente CH₃
- X⁻ représente CH₃SO₄⁻

### Estérification :

493 grammes d'acides gras de suif partiellement hydrogénés, d'indice d'iode 30, sont chargés en atmosphère inerte dans un réacteur en acier inox ; 150 g de triéthanolamine sont ajoutés sous agitation. Le mélange est chauffé au moins 12 heures à 140°C sous vide partiel pour éliminer l'eau de réaction. L'avancement de la réaction est suivi comme précédemment par un dosage acide/base qui détermine l'acidité résiduelle. 611 g d'un produit jaune, liquide à chaud, appelé "esteramine 2" sont récupérés, constitué essentiellement d'un mélange d'amine non estérifiée, mono, di et tri estérifiée.

### Quaternisation :

A 611 g d"'esteramine 2" sont ajoutés sous agitation 122 g de diméthylsulfate à une température de 60-80°C. Après une heure de digestion, l'absence quasi totale d'amine résiduelle est vérifiée par dosage acide/base. On obtient 733 g d'esteramine quaternisée. Le produit est ensuite dilué à 90 % par ajout de 11 % environ en poids d'isopropanol ; on obtient 815 g de produit appelé "quat n°2".

### EXEMPLE 3

Préparation du tensioactif appelé "quat n°3" constitué par un mélange de composés répondant à la formule I dans laquelle :
- n + n' + n" = 6
- p + q + r + x + y + z = 3
- Y₁, Y_{1'}, Y₂, Y_{2'}, Y₃, Y_{3'} sont des groupes oxy
- R₄ représente CH₃
- X⁻ représente CH₃SO₄⁻

### Estérification :

544 grammes d'acides gras de palme partiellement hydrogénés dont l'indice d'iode se situe vers 30 sont chargés en atmosphère inerte dans un réacteur en acier inox. 172 g de triéthanolamine sont ajoutés sous agitation. Le mélange est chauffé au moins 12 heures à 140°C comme dans l'exemple 1 sous vide partiel pour éliminer l'eau de réaction. L'avancement de la réaction est suivi par un dosage acide/base qui détermine l'acidité résiduelle. 680 g d'un produit jaune, liquide à chaud, appelé "esteramine 3" sont récupérés, constitué essentiellement d'un mélange d'amine non estérifiée, mono, di et tri estérifiée.

### Quaternisation :

A 680 g d"'esteramine 3" sont ajoutés sous agitation 140 g de diméthylsulfate à une température de 60-80°C. Après une heure de digestion, l'absence quasi totale d'amine résiduelle est vérifiée par dosage acide/base. On obtient 820 g d'esteramine quaternisée. Le produit est dilué à 90 % par ajout de 11 % environ en poids d'isopropanol ; on obtient 911 g de produit appelé "quat n°3".

### EXEMPLE 4

Préparation du tensioactif appelé "quat n°4" constitué par un mélange de composés répondant à la formule I dans laquelle :
- n + n' + n" = 6 ou 5
- p + q + r + x + y + z = 3 ou 1
- Y₁, Y_{1'}, Y₂, Y_{2'}, Y₃, Y_{3'} sont des groupes oxy ou NH
- R₄ représente CH₃
- X⁻ représente CH₃SO₄⁻

### Estérification et amidification :

594,5 grammes d'acides gras de palme partiellement hydrogénés dont l'indice d'iode se situe vers 30 sont chargés en atmosphère inerte dans un réacteur en acier inox. 172 g de triéthanolamine et 18,9 g de diméthylamino propylamine sont ajoutés sous agitation. Le mélange est chauffé au moins 12 heures à 145°C sous vide partiel pour éliminer l'eau de réaction. L'avancement de la réaction est suivi par un dosage acide/base qui détermine l'acidité résiduelle, comme dans l'exemple 1. 746,1 g d'un produit jaune, liquide à chaud, appelé "amine n°4" sont récupérés, constitué d'un mélange d'amines de départ, d'amines mono, di et tri estérifiées et d'alkylamidoamine.

### Quaternisation :

A 746,1 g de l'"amine n°4" décrite ci-dessus sont ajoutés sous agitation 162 g de diméthylsulfate à une température de 60-80°C. Après une heure de digestion, l'absence quasi totale d'amine résiduelle est vérifiée par dosage acide/base. On obtient 908,1 g de produit quaternisé qui est dilué à 90 % par ajout de 11 % environ en poids d'isopropanol. On obtient 1009 g de produit appelé "quat n°4".

### EXEMPLE 5

Préparation du tensioactif appelé "quat n°5" constitué par un mélange de composés répondant à la formule I dans laquelle :
- n + n' + n'' = 5
- p + q + r + x + y + z = 2 ou 1
- Y₁, Y_{1'}, Y₂, Y_{2'}, Y₃, Y_{3'} sont des groupes oxy ou NH
- R₄ représente CH₃
- X⁻ représente CH₃SO₄⁻

### Estérification et amidification :

544 grammes d'acides gras de palme partiellement hydrogénés dont l'indice d'iode se situe vers 30 sont chargés en atmosphère inerte dans un réacteur en acier inox. 137 g de diméthylamino propane 1,2 diol et 18,1 g de diméthylamino propylamine sont ajoutés sous agitation. Le mélange est chauffé au moins 14 heures à 145°C sous vide partiel pour éliminer l'eau de réaction. L'avancement de la réaction est suivi par un dosage acide/base qui détermine l'acidité résiduelle, comme dans l'exemple 1. 708,2 g d'un produit jaune, liquide à chaud, appelé "amine n°5" sont récupérés, constitué d'un mélangé d'amines de départ, d'amines mono, diestérifiées et d'amido amine.

### Quaternisation :

A 708,2 g de l'"amine n°5" décrite ci-dessus sont ajoutés sous agitation 162,2 g de diméthylsulfate à une température de 60-80°C. Après une heure de digestion, l'absence quasi totale d'amine résiduelle est vérifiée par dosage acide/base. On obtient 870,4 g de produit quaternisé qui est dilué à 90 % par ajout de 11 % environ en poids d'isopropanol. On obtient 967,1 g de produit appelé "quat n°5".

### EXEMPLE 6

Un mélange de 538 g d'huile de palme partiellement hydrogénée et de 150 g de triéthanolamine ou TEA est chauffé sous atmosphère d'azote pendant au moins 4 h à 140°C, en présence de 1,5 % de méthylate de sodium, jusqu'à une teneur en TEA libre inférieure à 6 % en poids.

Le milieu réactionnel est ensuite traité par 126 g de Me₂SO₄. Le mélange d'ammoniums quaternaires formés est dilué dans de l'alcool isopropylique pour donner un extrait sec de 80 à 85 %, puis décoloré à l'eau oxygénée ou un chlorite.

L'agent tensioactif contenu dans le mélange ainsi préparé répond statistiquement à la formule (I) dans laquelle :
- n + n'+ n" = 6
- p + q + r + x + y + z = 3
- Y₁, Y_{1'}, Y₂, Y_{2'}, Y₃, Y_{3'} sont des groupes oxy
- R₄ = CH₃
- X⁻ = CH₃SO₄

### EXEMPLE 7

Un mélange de 294 g d'huile de palme, de 5,1 g de diméthylaminopropylamine, de 73 g de triéthanolamine et 0,2 g d'acide phosphoreux est chauffé sous balayage d'azote à 140°C pour distiller l'eau de réaction formée. La température est progressivement augmentée à 190°C puis maintenue pendant au moins 4 h. A un taux de conversion minimum de 75 % de la triéthanolamine, le milieu réactionnel est refroidi à 60°C, puis quaternisé par 62 g de diméthylsulfate (DMS).

48 g d'isopropanol (IPA) sont ajoutés pour ajuster l'extrait sec à 90 %. La matière active finale, mesurée selon la norme AFNOR NFT 73-320 (ou ISO 2871-1 de mai 1989) est alors de 0,81 mE/g.

L'agent tensioactif contenu dans le mélange ainsi préparé répond statistiquement à la formule (I) dans laquelle :
- n + n' + n'' = 6 ou 5
- p + q + r + x + y + z = 3 ou 1
- Y₁, Y_{1'}, Y₂, Y_{2'}, Y₃, Y_{3'} sont des groupes oxy ou NH
- R₄ = CH₃
- X⁻ = CH₃SO₄

### EXEMPLE 8

Un mélange de 425 g d'huile de palme, de 110 g de diéthylamino-3 propanediol 1,2 est porté à 190°C sous agitation et atmosphère d'azote pendant 8 h.

A un taux de conversion minimum de 75 % de l'aminodiol, le milieu réactionnel est refroidi à 60°C puis quaternisé par 89 g de DMS. L'ensemble est chauffé à 80°C pendant 1 h.

La matière active finale, mesurée selon la norme NFT 73-320 est alors de 0,87 mE/g.

L'agent tensioactif contenu dans le mélange ainsi préparé répond à la formule (I) dans laquelle :
- n + n'+ n" = 7
- p + q + r + x + y + z = 2
- Y₁, Y_{1'}, Y₂, Y_{2'}, Y₃, Y_{3'} sont des groupes oxy
- R₄ = CH₃
- X⁻ = CH₃SO₄

Les Exemples 9 à 13 suivants mettent en lumière les avantages d'une base assouplissante de l'invention comprenant au moins un composé tensioactif répondant à la formule (I) et un solvant répondant à la formule (II).

### EXEMPLE 13

Assouplissant à 5 % d'agent actif préparé à partir de l'un des sels d'ammonium quaternaire n°1 à 5 obtenus selon l'invention.

Dans un mélangeur sous agitation, on introduit l'eau chaude à 40°C, puis le sel d'ammonium quaternaire à 50°C environ ; on agite jusqu'à homogénéisation complète. On refroidit à 30°C puis on ajoute, selon le besoin, le colorant, le parfum et un conservateur.

Les caractéristiques des compositions obtenues sont présentées dans le Tableau 5.

**TABLEAU 5**

| **% m/m** | **quat n°1** | **quat n°2** | **quat n°3** | **quat n°4** | **quat n°5** |
|---|---|---|---|---|---|
| % eau | 94,2 | 94,2 | 93,9 | 94,1 | 94,1 |
| % quat à 90% dans l'isopropanol | 5,6 | 5,6 | 5,6 | 5,6 | 5,6 |
| | | | | | |
| % parfum | 0,2 | 0,2 | 0,5 | 0,3 | 0,3 |
| caractéristiques | | | | | |
| | | | | | |
| pH tel que | 3,2 | 2,9 | 3,4 | 3,0 | 3,2 |
| Viscosité 20°C à temps 0 J | 131 | 120 | 60 | 65 | 57 |
| Viscosité 20°C après 6 jours à 50°C | 153 | 128 | 120 | 90 | 70 |
| Viscosité 20°C après 20 jours à 50°C | 145 | 157 | 122 | 95 | 68 |

Toutes les viscosités des exemples 13 à 15 sont mesurées à 20°C à l'aide d'un viscosimètre contraves TV MS R2.

### EXEMPLE 14

Assouplissant à 15 % ou 18 % d'agent actif préparé à partir de l'un des sels d'ammonium quaternaire n°1 à 4 obtenus selon l'invention.

Dans un mélangeur sous agitation, on introduit l'eau chaude à 50°C environ, puis le sel d'ammonium quaternaire à 50°C environ. On ajoute l'additif fluidifiant ; on refroidit à 30°C puis on ajoute, selon le besoin, le colorant, le parfum et un conservateur.

Les caractéristiques des compositions obtenues sont présentées dans le Tableau 6.

**TABLEAU 6**

| **% m/m** | **quat n°1** | **quat n°2** | **quat n°3** | **quat n°4** |
|---|---|---|---|---|
| % eau | qs | qs | qs | qs |
| % quat (90% matière active dans 10% d'isopropanol) | 16,7 | 16,7 | 20 | 20 |
| % CaCl₂ (fluidifiant) | 0,075 | 0,075 | 0,21 | 0,2 |
| | | | | |
| % parfum | 1,2 | 1,2 | 1,4 | 1,4 |
| | | | | |

| caractéristiques | | | | |
|---|---|---|---|---|
| | | | | |
| pH tel que | 3,2 | 3,1 | 2,9 | 3,0 |
| Viscosité 20°C à t = 0 jour | 43 | 36 | 36 | 42 |
| Viscosité 20°C après 6 jours à 50°C | 37 | 34 | 42 | 45 |
| Viscosité à 20°C après 20 jours à 50°C | 38 | 52 | 48 | 53 |

### EXEMPLE 15

Assouplissant à 20 % d'agent actif préparé à partir de l'un des sels d'ammonium quaternaire n°1, 3 et 5 obtenus selon l'invention.

Dans un mélangeur sous agitation, on introduit l'eau à 50°C environ, puis le sel d'ammonium quaternaire à 50°C environ. On agite jusqu'à homogénéisation complète ; on refroidit à 35-38°C. On ajoute l'additif fluidifiant puis on ajoute, selon le besoin, le colorant, le parfum et un conservateur.

Les caractéristiques des compositions obtenues sont présentées dans le Tableau 7.

**TABLEAU 7**

| **% m/m** | **quat n°1** | **quat n°3** | **quat n°5** |
|---|---|---|---|
| eau | qsp | | |
| quat (à 90% dans l'isopropanol | 22,22 | 22,25 | 22,25 |
| | | | |
| | | | |
| quat TEA/DMS * (fluidifiant) | 0,7 | 0 | 0,8 |
| CaCl₂ (fluidifiant) | 0 | 0,24 | 0 |
| | | | |
| Parfum | 0,8 | 1,4 | 0,9 |
| | | | |

| Caractéristiques | | | |
|---|---|---|---|
| | | | |
| pH tel que | 2,9 | 3,5 | 3,4 |
| | | | |
| Viscosité à 20°C | 56 | 46 | 110 |
| Viscosité après 6 jours à 50°C | 56 | 62 | 180 |
| Viscosité après 20 jours à 50°C | 56 | 100 | 170 |

| | | | |
|---|---|---|---|
| * TEA/DMS est le sel d'ammonium quaternaire obtenu par réaction d'une mole de sulfate de diméthyle sur une mole de triéthanolamine. | | | |

## Revendications

1. Composé tensioactif cationique comprenant, sous forme de sel d'ammonium quaternaire, un produit de condensation entre, d'une part, une fraction d'acides gras saturés ou insaturés, linéaires ou ramifiés, ou de dérivés desdits acides, lesdits acides gras ou dérivés présentant chacun une chaîne hydrocarbonée dont le nombre d'atomes est compris entre 5 et 23, et d'autre part, au moins une amine tertiaire fonctionnalisée, les chaînes alkyle de ladite amine tertiaire comportant ensemble au moins quatre atomes de carbone, **caractérisé en ce que** le rapport molaire fraction d'acides gras/amine tertiaire est compris entre 1,85 et 1,40, lesdites chaînes alkyle de l'amine tertiaire comportant ensemble jusqu'à 12 atomes de carbone et comportant ensemble de une à quatre fonctions choisies chacune dans le groupe comprenant les fonctions hydroxyle et amine.

2. Composé selon la revendication 1, caractérisé en ce que lesdits acides gras sont sous forme libre et lesdits dérivés sont choisis parmi les esters, anhydrides et chlorures d'acides desdits acides gras.

3. Composé selon la revendication 1 caractérisé en ce que lesdits acides gras sont sous forme d'esters de glycérol et de préférence de triesters de glycérol.

4. Composé tensioactif selon la revendication 1, caractérisé en ce que, la fraction d'acides gras comporte au moins 50 % en moles d'acides linéaires en C₁₆ à C₁₈.

5. Composé selon la revendication 4, caractérisé en ce que la fraction d'acides gras C₁₆/C₁₈ comporte majoritairement des acides linéaires en C₁₈, et le rapport molaire fraction d'acides gras/amine tertiaire est compris entre 1,82 et 1,60.

6. Composé selon la revendication 4, caractérisé en ce que la fraction d'acides gras C₁₆/C₁₈ comporte majoritairement des acides linéaires en C₁₆, et le rapport molaire fraction d'acides gras/amine tertiaire est compris entre 1,85 et 1,66.

7. Composé selon la revendication 4 ou 5, caractérisé en ce que la fraction d'acides gras C₁₆/C₁₈ comporte majoritairement des acides linéaires en C₁₈, et cette fraction présente une insaturation globale, exprimée par l'indice d'iode, comprise entre 10 et 33, et notamment entre 15 et 33.

8. Composé selon la revendication 4 ou 6, caractérisé en ce que la fraction d'acides gras C₁₆/C₁₈ comporte majoritairement des acides linéaires en C₁₆, et cette fraction présente une insaturation globale, exprimée par l'indice d'iode, comprise entre 10 et 43, et notamment entre 15 et 35.

9. Composé selon la revendication 1, caractérisé en ce qu'il est un produit de condensation d'une fraction d'acides gras et d'un mélange d'une amine tertiaire polyhydroxylée, et d'une amine tertiaire substituée par une fonction hydroxyle ou amine primaire ou secondaire, dans un rapport molaire de la première par rapport à la seconde au moins égal à 2.

10. Composé selon la revendication 9, caractérisé en ce que la fraction d'acides gras C₁₆/C₁₈ comporte majoritairement des acides linéaires en C₁₆, et cette fraction présente une insaturation globale, exprimée par un indice d'iode inférieur à 35.

11. Composé selon la revendication 9, caractérisé en ce que la fraction d'acides gras C₁₆/C₁₈ comporte majoritairement des acides linéaires en C₁₈, et cette fraction présente une insaturation globale, exprimée par un indice d'iode inférieur à 33.

12. Composé tensioactif cationique répondant à la formule (I) suivante : 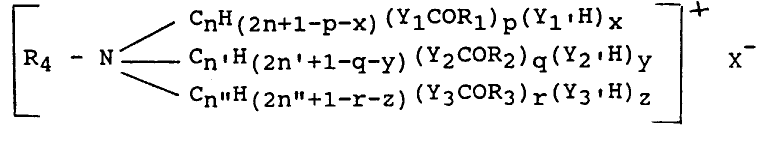 dans laquelle :
- n, n', n'' sont des nombres entiers non nuls dont la moyenne de la somme est au moins égale à 4, rapportée à une mole de tensioactif,
- p, q, r, x, y, z sont des nombres entiers ou zéro,
- Y₁, Y_{1'}, Y₂, Y_{2'}, Y₃, Y_{3'} sont des groupes fonctionnels,
- R₁, R₂, R₃ sont respectivement une longue chaîne hydrocarbonée alkyle ou alkényle, ayant chacune de 5 à 23 atomes de carbone,
- R₄ est une chaîne alkyle ayant de 1 à 5 atomes de carbone, et
- X est un contre-ion,
caractérisé en ce que :
- rapportée à une mole, la moyenne de la somme de p, q et r est comprise statistiquement entre 1,85 et 1,40,
rapportée à une mole, la moyenne de la somme de n, n', n'' est statistiquement au plus égale à 12, et
les groupes fonctionnnels Y₁, Y_{1'}, Y₂, Y_{2'}, Y₃, Y_{3'} étant choisis chacun parmi les groupes oxy, N et NH, et étant ensemble en nombre compris entre 1 et 4.

13. Composé tensioactif selon la revendication 12, caractérisé en ce que Y₁, Y_{1'}, Y₂, Y_{2'}, Y₃, Y_{3'} sont des groupes oxy.

14. Base assouplissante, caractérisée en ce qu'elle comprend au moins un composé tensioactif selon l'une quelconque des revendications 1 à 13 en solution dans un solvant.

15. Base assouplissante, selon la revendication 14, caractérisée en ce qu'elle comprend un mélange de composés tensioactifs homologues, indépendamment de la nature des acides gras, répondant chacun à la définition selon l'une quelconque des revendications 1 à 13.

16. Base selon la revendication 14, caractérisée en ce qu'elle comprend à titre de solvant, au moins un composé répondant à la formule II suivante:
(R₅ - E)ₘ- Cⱼ H_{2(j+1)-m-2d+i} Oₖ₊ᵢ
dans laquelle :
- R₅ est une longue chaîne hydrocarbonée saturée ou insaturée, ayant de 5 à 23 atomes de carbone
- E est une fonction 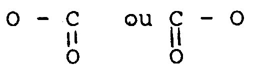
- m est un nombre entier compris entre 1 et 8
- j est un nombre entier compris entre 1 et 36
- k est un nombre entier ou zéro
- i est un nombre entier ou zéro
- d est égal à 0, 1 ou 2.

17. Base selon la revendication 16, caractérisée en ce que i est égal à 0.

18. Base selon la revendication 17, caractérisée en ce que si m est égal à 1, j est compris entre 1 et 18 et de préférence entre 1 et 4, et k et d sont égaux à 0.

19. Base selon la revendication 18, caractérisée en ce que si j est égal à 1, R₅ est une chaîne hydrocarbonée ayant de 7 à 17 atomes de carbone.

20. Base selon la revendication 17, caractérisée en ce que si m est égal à 2, k et d sont égaux à 0 et j est compris entre 2 et 12.

21. Base selon la revendication 17, caractérisée en ce que si m est égal à 2, k est égal à 1, j est compris entre 4 et 12, d est égal à 0, et R₅ est une chaîne hydrocarbonée ayant 7 ou 9 atomes de carbone.

22. Base selon la revendication 17, caractérisée en ce que m est égal à 3, j est égal à 3 ou 4, k et d sont égaux à 0 et R₅ est une chaîne hydrocarbonée ayant 7 ou 9 atomes de carbone.

23. Base selon la revendication 17, caractérisée en ce que si m est égal à 3, j est égal à 6 et k et d sont égaux à 0.

24. Base selon la revendication 17, caractérisée en ce que si m est égal à 4, j est égal à 5 et k et d sont égaux à 0.

25. Base selon l'une quelconque des revendications 16 à 24, caractérisée en ce qu'elle comprend 60 à 99% en poids d'au moins un tensioactif selon la revendication 12 ou 13 et 40 à 1% en poids d'au moins un composé répondant à la formule (II).

26. Composition assouplissante, caractérisée en ce qu'elle comprend une base selon l'une quelconque des revendications 14 à 25 et éventuellement des additifs.

27. Composition selon la revendication 26, caractérisée en ce qu'elle comprend au moins un composé tensioactif selon l'une quelconque des revendications 2 à 13, la concentration pondérale en dit tensioactif étant comprise entre 3 et 30% du poids de ladite composition.

28. Composition selon la revendication 26, caractérisée en ce qu'elle comprend au moins un composé tensioactif selon la revendication 3, la concentration pondérale en dit tensioactif représentant au moins 60% en poids de ladite composition.

29. Composition selon la revendication 28, caractérisée en ce que le solde de ladite composition comprend des produits secondaires susceptibles d'être obtenus par la réaction de condensation entre un triester de glycérol et une amine tertiaire, et notamment des monoglycérides, des diglycérides, du glycérol.

30. Composition selon la revendication 28, caractérisée en ce que le solde de ladite composition comprend des produits secondaires susceptibles d'être obtenus par la réaction de quaternisation du produit de condensation, dont ledit produit non quaternisé.

31. Procédé pour préparer directement un mélange de tensioactifs selon la revendication 12, selon lequel on fait réagir des acides gras répondant respectivement aux formules R₁COOH, R₂COOH, R₃COOH, dans lesquelles R₁, R₂, R₃ sont respectivement une longue chaîne hydrocarbonée alkyle ou alkényle, linéaire ou ramifiée, ayant de 5 à 23 atomes de carbone, ou leurs dérivés comprenant leurs esters, anhydrides et chlorures d'acide, avec au moins une amine tertiaire répondant à la formule (III) suivante : 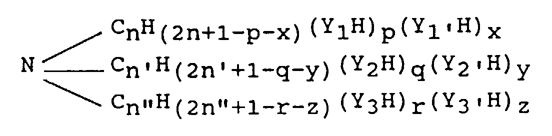 dans laquelle :
- n, n', n'' sont des nombres entiers non nuls
- p, q, r, x, y, z sont des nombres entiers ou zéro
- rapportée à une mole, la moyenne de la somme de n, n' et n'' est au moins égale à 4
- Y₁, Y_{1'}, Y₂, Y_{2'}, Y₃, Y_{3'} sont des groupes fonctionnels
puis on fait réagir le produit de condensation ainsi obtenu avec un agent alkylant, en présence ou non d'un solvant
caractérisé en ce que les acides gras sont sous forme libre et leur concentration molaire est comprise entre 1,85 et 1,40 fois la concentration molaire globale en amines tertaires répondant chacune à la formule (III) dans laquelle, en outre :
- la somme de n, n' et n'' est comprise entre 4 et 12,
- la somme de p, q, r, x, y et z est en moyenne statistique comprise entre 1 et 4,
- Y₁, Y_{1'}, Y₂, Y_{2'}, Y₃, Y_{3'} sont choisis chacun parmi les groupes oxy, N et NH.

32. Procédé pour préparer directement une composition assouplissante selon la revendication 28, selon lequel on fait réagir des acides gras répondant respectivement aux formules R₁COOH, R₂COOH, R₃COOH, dans lesquelles R₁, R₂, R₃ sont respectivement une longue chaîne hydrocarbonée alkyle ou alkényle ayant de 5 à 23 atomes de carbone, lesdits acides gras étant sous forme de triesters de glycérides avec au moins une amine tertiaire répondant à la formule (III) suivante : 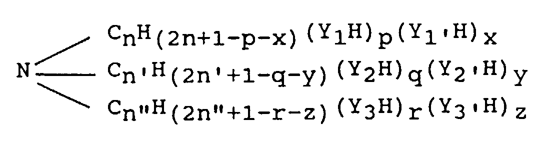 dans laquelle :
- n, n', n'' sont des nombres entiers non nuls
- p, q, r, x, y, z sont des nombres entiers ou zéro
- rapportée à une mole, la moyenne de la somme de n, n' et n'' est au moins égale à 4
- Y₁, Y_{1'}, Y₂, Y_{2'}, Y₃, Y_{3'} sont des groupes fonctionnels
puis on fait réagir le produit de condensation ainsi obtenu avec un agent alkylant, en présence ou non d'un solvant
caractérisé en ce que
- rapportée à une mole, la moyenne de la somme de n, n' et n'' est comprise statistiquement entre 4 et 12,
- rapportée à une mole, la moyenne de la somme de p, q, r, x, y et z est comprise statistiquement entre 1 et 4,
- Y₁, Y_{1'}, Y₂, Y_{2'}, Y₃, Y_{3'} sont choisis chaucun parmi les groupes oxy, N et NH,
et en ce qu'on fait réagir partiellement l'amine tertiaire, selon une transestérification et/ou transestéramidification, avec le triester de glycérol.

33. Procédé selon la revendication 32, caractérisé en ce que le rapport molaire du triester de glycérol à l'amine tertiaire est compris entre 1/3 et 1.

34. Procédé selon l'une quelconque des revendications 31 à 33, caractérisé en ce que l'agent alkylant est choisi parmi les halogénures, les sulfates, les phosphates et carbonates d'alkyle.

35. Procédé selon la revendication 32, caractérisé en ce qu'on fait aussi réagir partiellement le produit de condensation avec l'agent alkylant.

36. Procédé selon la revendication 32, caractérisé en ce que l'amine tertiaire est monofonctionnelle, c'est-à-dire comporte une seule fonction hydroxyle ou amine primaire ou secondaire, et est notamment choisie dans le groupe comprenant :
la N,N-diméthylaminopropylamine
la N,N-diéthylaminopropylamine
la N,N-diméthylaminoéthanolamine
la N,N-diéthylaminoéthanolamine
la N,N-diméthylaminoisopropanolamine
la N,N-diéthylaminoisopropanolamine.

37. Procédé selon la revendication 36, caractérisé en ce qu'au moins une amine tertiaire polyfonctionnelle, c'est-à-dire comportant plusieurs fonctions hydroxyle ou amine primaire ou secondaire, est associée à l'amine tertiaire monofonctionnnelle, dans un rapport molaire amine tertiaire polyfonctionnelle/amine tertiaire monofonctionnelle compris entre 2 et 200.

38. Procédé selon la revendication 37, caractérisé en ce que l'amine tertiaire polyfonctionnelle est choisie parmi le N,N diéthylamino-2,3 propane diol, le N,N diméthylamino-2,3 propane diol, la méthyldiéthanolamine, l'éthyldiéthanolamine, et la triéthanolamine.

## Patentansprüche

1. Kationische, tensioaktive Verbindung, die, in Form eines quartären Ammoniumsalzes, ein Kondensationsprodukt aus einerseits einer Fraktion an gesättigten oder ungesättigten, geradkettigen oder verzweigten Fettsäuren oder Derivate der genannten Fettsäuren, wobei die Fettsäuren bzw. die Derivate jeweils eine Kohlenwasserstoffkette mit 5 bis 23 Kohlenstoffatomen aufweisen und andererseits zumindest einem funktionalisiertem tertiären Amin enthält, wobei die Alkylketten des genannten tertiären Amins zusammen mindestens vier Kohlenstoffatome aufweisen, dadurch gekennzeichnet, daß das molare Verhältnis der Fraktion an Fettsäuren/tertiärem Amin im Bereich von 1,85 bis 1,40 liegt, daß die Alkylketten des tertiären Amins zusammen bis zu 12 Kohlenstoffatome aufweisen und zusammen eine bis vier funktionelle Gruppen, ausgewählt aus der Gruppe der Hydroxyl- und Aminfunktionen, aufweisen.

2. Verbindung nach Anspruch 1, dadurch gekennzeichnet, daß die Fettsäuren in freier Form vorliegen, und daß die Derivate ausgewählt sind aus den Estern, den Anhydriden und den Säurechloriden der genannten Fettsäuren.

3. Verbindung nach Anspruch 1, dadurch gekennzeichnet, daß die Fettsäuren in Form von Glycerinestern und vorzugsweise als Triester des Glycerins vorliegen.

4. Tensioaktive Verbindung nach Anspruch 1, dadurch gekennzeichnet, daß die Fraktion der Fettsäuren zumindest 50 Mol.-% geradkettige C₁₆ bis C₁₈-Säuren aufweist.

5. Verbindung nach Anspruch 4, dadurch gekennzeichnet, daß die C₁₆/C₁₈-Fettsäurefraktion in überwiegendem Anteil geradkettige C₁₈-Säuren aufweist, und daß das molare Verhältnis der Fraktion an Fettsäuren/tertiäres Amin im Bereich von 1,82 bis 1,60 liegt.

6. Verbindung nach Anspruch 4, dadurch gekennzeichnet, daß die Fraktion der C₁₆/C₁₈-Fettsäuren im überwiegenden Anteil geradkettige C₁₆-Säuren aufweist, und daß das molare Verhältnis der Fraktion an Fettsäuren/tertiärem Amin im Bereich von 1,85 bis 1,66 liegt.

7. Verbindung nach Anspruch 4 oder 5, dadurch gekennzeichnet, daß die Fraktion der C₁₆/C₁₈-Fettsäuren im überwiegenden Anteil geradkettige C₁₈-Säuren aufweist, und daß diese Fraktion eine Gesamtunsättigung, ausgedrückt durch den Iodindex, von 10 bis 33, insbesondere von 15 bis 33, aufweist.

8. Verbindung nach Anspruch 4 oder 6, dadurch gekennzeichnet, daß die Fraktion der C₁₆/C₁₈-Fettsäuren im überwiegenden Anteil geradkettige C₁₆-Säuren aufweist, und daß diese Fraktion eine Gesamtunsättigung, ausgedrückt durch den Iodindex, von 10 bis 43, und insbesondere von 15 bis 35 aufweist.

9. Verbindung nach Anspruch 1, dadurch gekennzeichnet, daß sie ein Kondensationsprodukt aus einer Fraktion an Fettsäuren und einer Mischung eines tertiären polyhydroxylierten Amins und eines durch eine Hydroxylfunktion oder ein primäres oder sekundäres Amin substituiertes tertiäres Amin ist, und zwar in einem molaren Verhältnis der ersten zu der zweiten von zumindest 2.

10. Verbindung nach Anspruch 9, dadurch gekennzeichnet, daß die Fraktion der C₁₆/C₁₈-Fettsäuren im überwiegenden Anteil geradkettige C₁₆-Säuren aufweist, und daß diese Fraktion eine Gesamtunsättigung, ausgedrückt durch den Iodindex, kleiner 35 aufweist.

11. Verbindung nach Anspruch 9, dadurch gekennzeichnet, daß die Fraktion der C₁₆/C₁₈-Fettsäuren im überwiegenden Anteil geradkettige C₁₈-Säuren aufweist, und daß diese Fraktion eine Gesamtunsättigung, ausgedrückt durch den Iodindex, kleiner 33 aufweist.

12. Kationische tensioaktive Verbindung, die der folgenden Formel (I) entspricht: 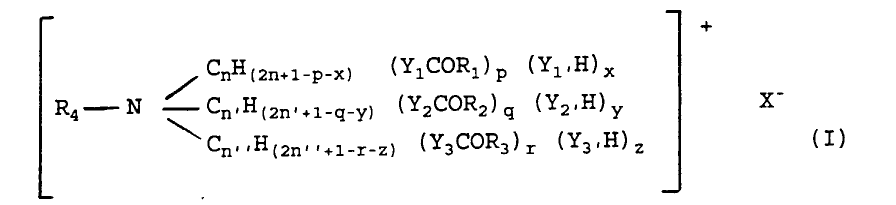 in der
- n, n', n" ganze Zahlen, jedoch nicht Null bedeuten, deren mittlerer Summenwert, bezüglich einem Mol an Tensioaktiv, zumindest 4 beträgt,
- p, q, r, x, y, z ganze Zahlen oder Null bedeuten,
- Y₁, Y_{1'}, Y₂, Y_{2'}, Y₃, Y_{3'} funktionelle Gruppen sind,
- R₁, R₂, R₃ jeweils eine lange Alkyl- oder Alkylenkohlenwasserstoffkette mit jeweils 5 bis 23 Kohlenstoffatomen ist,
- R₄ eine Alkylkette mit 1 bis 5 Kohlenstoffatomen ist, und
- X ein Gegenion ist,
dadurch gekennzeichnet,
- daß bezüglich eines Moles der mittlere Summenwert von p, q und r statistisch im Bereich von 1,85 bis 1,40 liegt, bezüglich eines Moles, der mittlere Summenwert von n, n', n" statistisch höchstens gleich 12 beträgt, und daß die funktionellen Gruppen Y₁, Y_{1'}, Y₂, Y_{2'}, Y₃, Y_{3'} jeweils ausgewählt sind aus den Gruppen Oxy, N und NH, und zusammen in einer Zahl von 1 bis 4 vorhanden sind.

13. Tensioaktive Verbindung nach Anspruch 12, dadurch gekennzeichnet, daß Y₁, Y_{1'}, Y₂, Y_{2'}, Y₃, Y_{3'} Oxy-Gruppen sind.

14. Weichmacherbasismittel, dadurch gekennzeichnet, daß es zumindest eine tensioaktive Verbindung nach einem der Ansprüche 1 bis 13 in Lösung in einem Lösungsmittel aufweist.

15. Weichmacherbasismittel nach Anspruch 14, dadurch gekennzeichnet, daß es, unabhängig von der Natur der Fettsäuren, eine Mischung an homologen tensioaktiven Verbindungen enthält, die jeweils der Definition nach einem der Ansprüchem 1 bis 13 entsprechen.

16. Basismittel nach Anspruch 14, dadurch gekennzeichnet, daß es als Lösungsmittel zumindest eine Verbindung enthält, die der folgenden Formel (II) entspricht:
(R₅ - E)ₘ - Cⱼ H_{2(j+1)-m-2d+i} Oₖ₊ᵢ (II)
in der
- R₅ eine lange gesättigte oder ungesättigte Kohlenwasserstoffkette ist, die 5 bis 23 Kohlenstoffatome aufweist,
- E eine Funktion 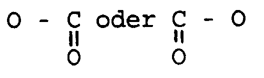 ist,
- m eine ganze Zahl im Bereich von 1 bis 8 ist,
- j eine ganze Zahl im Bereich von 1 bis 36 ist,
- k eine ganze Zahl oder Null ist,
- i eine ganze Zahl oder Null ist,
- d Null, 1 oder 2 ist.

17. Basismittel nach Anspruch 16, dadurch gekennzeichnet, daß i gleich Null ist.

18. Basismittel nach Anspruch 17, dadurch gekennzeichnet, daß, falls m gleich 1 ist, j im Bereich von 1 bis 18 liegt und vorzugsweise im Bereich von 1 bis 4, und daß k und d gleich Null sind.

19. Basismittel nach Anspruch 18, dadurch gekennzeichnet, daß, falls j gleich 1 ist, R₅ eine Kohlenwasserstoffkette mit 7 bis 17 Kohlenstoffatomen ist.

20. Basismittel nach Anspruch 17, dadurch gekennzeichnet, daß, falls m gleich 2 ist, k und d gleich Null sind, und j im Bereich von 2 bis 12 liegt.

21. Basismittel nach Anspruch 17, dadurch gekennzeichnet, daß, falls m gleich 2 ist, k gleich 1 ist, j im Bereich von 4 bis 12 liegt, d gleich Null ist, und R₅ eine Kohlenwasserstoffkette mit 7 oder 9 Kohlenstoffatomen ist.

22. Basismittel nach Anspruch 17, dadurch gekennzeichnet, daß, falls m gleich 3 ist, j gleich 3 oder 4 ist, k und d gleich Null sind, und R₅ eine Kohlenwasserstoffkette mit 7 oder 9 Kohlenstoffatomen ist.

23. Basismittel nach Anspruch 17, dadurch gekennzeichnet, daß, falls m gleich 3 ist, j gleich 6 ist, und k und d gleich Null sind.

24. Basismittel nach Anspruch 17, dadurch gekennzeichnet, daß, falls m gleich 4 ist, j gleich 5 ist, und k und d gleich Null sind.

25. Basismittel nach einem der Ansprüche 16 bis 24, dadurch gekennzeichnet, daß es 60 bis 99 Gew.-% zumindest eines Tensioaktivs nach Anspruch 12 oder 13 und 40 bis 1 Gew.-% zumindest einer Verbindung der Formel (II) enthält.

26. Weichmacherzusamnensetzung, dadurch gekennzeichnet, daß sie ein Basismittel nach einem der Ansprüche 14 bis 25 und ggf. Zusätze aufweist.

27. Zusammensetzung nach Anspruch 26, dadurch gekennzeichnet, daß sie zumindest eine tensioaktive Verbindung nach einem der Ansprüche 2 bis 13 enthält, wobei die gewichtsbezogene Konzentration des genannten Tensioaktivs im Bereich von 3 bis 30 Gew.-% der genannten Zusammensetzung beträgt.

28. Zusammensetzung nach Anspruch 26, dadurch gekennzeichnet, daß sie zumindest eine tensioaktive Verbindung nach Anspruch 3 enthält, wobei die in gewichtsbezogene Konzentration des genannten Tensioaktivs zumindest 60 Gew.-% der Zusammensetzung darstellt.

29. Zusammensetzung nach Anspruch 28, dadurch gekennzeichnet, daß der Rest der Zusammensetzung Sekundärprodukte enthält, die durch die Kondensationsreaktion zwischen einem Glycerin triester und einem tertiären Amin erhalten werden, und insbesondere Monoglyceride, Diglyceride und Glycerin.

30. Zusammensetzung nach Anspruch 28, dadurch gekennzeichnet, daß der Rest der Zusammensetzung Sekundärprodukte enthält, die bei der Quarternisierungsreaktion des Kondensationsproduktes erhalten werden, wobei das Produkt nicht quarternisiert ist.

31. Verfahren zum direkten Herstellen einer Mischung an Tensioaktiven nach Anspruch 12, bei dem man Fettsäuren, die den Formeln R₁COOH, R₂COOH, R₃COOH entsprechen, in denen R₁, R₂, R₃ jeweils eine lange geradkettige oder verzweigte Alkyl- oder Alkylenkohlenwasserstoffkette mit 5 bis 23 Kohlenstoffatomen oder deren Derivate, die deren Ester, Anhydride oder Säurechloriden enthalten, bedeuten, mit zumindest einem tertiären Amin reagieren läßt, das der folgenden Formel (III) entspricht: 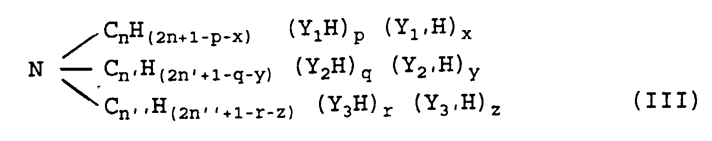 in der
- n, n', n" ganze Zahlen, jedoch nicht Null bedeuten
- p, q, r, x, y, z ganze Zahlen oder Null bedeuten
- bezüglich eines Mols, der mittlere Summenwert von n, n' und n" zumindest 4 ist,
- Y₁, Y_{1'}, Y₂, Y_{2'}, Y₃, Y_{3'} funktionelle Gruppen sind, man anschließend das so erhaltene Kondensationsprodukt
mit einem Alkylierungsagens in Gegenwart oder ohne ein Lösungsmittel reagieren läßt,
dadurch gekennzeichnet, daß die Fettsäuren in freier Form vorliegen und daß ihre molare Konzentration im Bereich von 1,85 bis 1,40 bezüglich der molaren Gesamtkonzentration an tertiären Aminen beträgt, die jeweils der Formel (III) entsprechen, in der darüber hinaus:
- die Summe von n, n' und n" im Bereich von 4 bis 12 liegt,
- die Summe von p, q, r, x, y und z im statistischen Mittel im Bereich von 1 bis 4 liegt,
- Y₁, Y_{1'}, Y₂, Y_{2'}, Y₃, Y_{3'} jeweils ausgewählt sind aus den Gruppen Oxy, N und NH.

32. Verfahren zum direkten Herstellen einer Weichmacherzusammensetzung nach Anspruch 28, bei dem man Fettsäuren, die jeweils den Formeln R₁COOH, R₂COOH, R₃COOH entsprechen, in denen R₁, R₂, R₃ jeweils eine lange Alkyl- oder Alkylenkohlenwasserstoffkette mit 5 bis 23 Kohlenstoffatomen bedeuten, wobei die genannten Fettsäuren in Form der Glycerintriester vorliegen mit zumindest einem tertiären Amin reagieren läßt, das der folgenden Formel (III) entspricht, reagieren läßt: 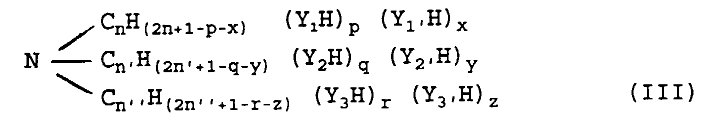 in der
- n, n', n" ganze Zahlen, jedoch nicht Null bedeuten,
- p, q, r, x, y, z ganze Zahlen oder Null bedeuten,
- bezüglich eines Mols, der mittlere Summenwert von n, n' und n" zumindest 4 beträgt,
- Y₁, Y_{1'}, Y₂, Y_{2'}, Y₃, Y_{3'} funktionelle Gruppen sind,
man anschließend das so erhalten Kondensationsprodukt mit einem Alkylierungsagens in Gegenwart oder ohne ein Lösungsmittel reagieren läßt,
dadurch gekennzeichnet, daß
- bezüglich eines Mols, der mittlere Summenwert von n, n' und n" statistisch im Bereich von 4 bis 12 liegt,
- bezüglich eines Mols, der mittlere Summenwert von p, q, r, x, y und z statistisch im Bereich von 1 bis 4 liegt,
- Y₁, Y_{1'}, Y₂, Y_{2'}, Y₃, Y_{3'} ausgewählt sind aus den Gruppen Oxy, N und NH,
und daß man das tertiäre Amin partiell entsprechend einer Transesterifizierung und/oder einer Transesteramidierung mit dem Triester von Glycerin reagieren läßt.

33. Verfahren nach Anspruch 32, dadurch gekennzeichnet, daß das molare Verhältnis des Glycerintriesters zum tertiären Amin im Bereich von 1/3 bis 1 liegt.

34. Verfahren nach einem der Ansprüche 31 bis 33, dadurch gekennzeichnet, daß das Alkylierungsagens ausgewählt ist aus den Alkylhalogeniden, den Alkylsulfaten, den Alkylphosphaten und den Alkylkarbonaten.

35. Verfahren nach Anspruch 32, dadurch gekennzeichnet, daß man das Kondensationsprodukt auch partiell mit dem Alkylierungsagens reagieren läßt.

36. Verfahren nach Anspruch 32, dadurch gekennzeichnet, daß das tertiäre Amin monofunktionell ist, d.h. daß es eine einzige primäre oder sekundäre Hydroxyl- oder Aminfunktion enthält, und daß es insbesondere ausgewählt ist aus der Gruppe bestehend aus:
N,N-Dimethylaminopropylamin
N,N-Diethylaminopropylamin
N,N-Dimethylaminoethanolamin
N,N-Diethylaminoethanolamin
N,N-Dimethylaminoisopropanolamin
N,N-Diethylaminoisopropanolamin

37. Verfahren nach Anspruch 36, dadurch gekennzeichnet, daß zumindest ein polyfunktionelles tertiäres Amin, d.h., eines das zahlreiche primäre oder sekundäre Hydroxyl- oder Aminfunktionen erhält, mit einem monofunktionellen tertiären Amin assoziiert wird, und zwar in einem molaren Verhältnis von polyfunktionellem tertiärem Amin/monofunktionellem tertiärem Amin im Bereich von 2 bis 200.

38. Verfahren nach Anspruch 37, dadurch gekennzeichnet, daß das polyfunktionelle tertiäre Amin ausgewählt ist aus N,N-Diethylamino-2,3-propandiol, N,N-Dimethylamino-2,3-propandiol, Methyldiethanolamin, Ethyldiethanolamin und Triethanolamin.

## Claims

1. Cationic surfactant compound comprising, in the form of a quaternary ammonium salt, a product of condensation between, on the one hand a fraction of saturated or unsaturated, linear or branched fatty acids, or of derivatives of said acids, said fatty acids or derivatives each possessing a hydrocarbon chain in which the number of atoms is between 5 and 23, and on the other hand at least one functionalized tertiary amine, the alkyl chains of said tertiary amine together containing at least four carbon atoms, characterized in that the fatty acid fraction/tertiary amine mole ratio is between 1.85 and 1.40, said alkyl chains of the tertiary amine together containing up to 12 carbon atoms and together containing from one to four functions each chosen from the group comprising hydroxyl and amine functions.

2. Compound according to Claim 1, characterized in that said fatty acids are in free form and said derivatives are chosen from the esters, anhydrides and acid chlorides of said fatty acids.

3. Compound according to Claim 1, characterized in that said fatty acids are in the form of glycerol esters, and preferably glycerol triesters.

4. Surfactant compound according to Claim 1, characterized in that the fatty acid fraction contains at least 50 mol% of linear C₁₆ to C₁₈ acids.

5. Compound according to Claim 4, characterized in that the C₁₆/C₁₈ fatty acid fraction predominantly contains linear C₁₈ acids, and the fatty acid fraction/tertiary amine mole ratio is between 1.82 and 1.60.

6. Compound according to Claim 4, characterized in that the C₁₆/c₁₈ fatty acid fraction predominantly contains linear C₁₆ acids, and the fatty acid fraction/tertiary amine mole ratio is between 1.85 and 1.66.

7. Compound according to Claim 4 or 5, characterized in that the C₁₆/C₁₈ fatty acid fraction predominantly contains linear C₁₈ acids, and this fraction possesses an overall unsaturation expressed by the iodine number of between 10 and 33, and in particular between 15 and 33.

8. Compound according to Claim 4 or 6, characterized in that the C₁₆/C₁₈ fatty acid fraction predominantly contains linear C₁₆ acids, and this fraction possesses an overall unsaturation expressed by the iodine number of between 10 and 43, and in particular between 15 and 35.

9. Compound according to Claim 1, characterized in that it is a product of condensation of a fatty acid fraction and of a mixture of a polyhydroxylated tertiary amine and a tertiary amine substituted with a hydroxyl or primary or secondary amine function, in a mole ratio of the first amine to the second equal to at least 2.

10. Compound according to Claim 9, characterized in that the C₁₆/C₁₈ fatty acid fraction predominantly contains linear C₁₆ acids, and this fraction possesses an overall unsaturation expressed by an iodine number of less than 35.

11. Compound according to Claim 9, characterized in that the C₁₆/C₁₈ fatty acid fraction predominantly contains linear C₁₈ acids, and this fraction possesses an overall unsaturation expressed by an iodine number of less than 33.

12. Cationic surfactant compound corresponding to the following formula (I): 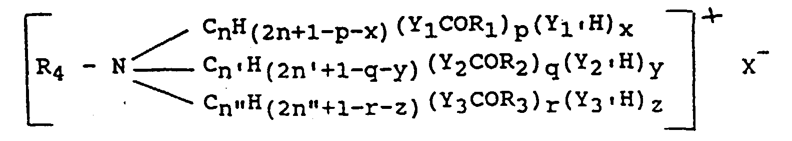 in which:
- n, n' and n" are non-zero integers, the mean of whose sum is equal to at least 4, expressed with reference to one mol of surfactant,
- p, q, r, x, y, z are integers or zero,
- Y₁, Y_{1'}, Y₂, Y_{2'}, Y₃, Y_{3'} are functional groups,
- R₁, R₂, R₃ are, respectively, a long alkyl or alkenyl hydrocarbon chain each having from 5 to 23 carbon atoms,
- R₄ is an alkyl chain having from 1 to 5 carbon atoms, and
- X is a counterion
characterized in that:
- expressed with reference to one mol, the mean of the sum of p, q and r is statistically between 1.85 and 1.40,
expressed with respect to one mol, the mean of the sum of n, n', n" is statistically equal to not more than 12, and
the functional groups Y₁, Y_{1'}, Y₂, Y_{2'}, Y₃, Y_{3'} each being chosen from oxy, N and NH groups, and numbering together between 1 and 4.

13. Surfactant compound according to Claim 12, characterized in that the Y₁, Y_{1'}, Y₂, Y_{2'}, Y₃, Y_{3'} are oxy groups.

14. Softening base, characterized in that it comprises at least one surfactant compound according to any one of Claims 1 to 13, dissolved in a solvent.

15. Softening base according to Claim 14, characterized in that it comprises a mixture of homologous surfactant compounds, independently of the nature of the fatty acids, each corresponding to the definition according to any one of Claims 1 to 13.

16. Base according to Claim 14, characterized in that it comprises as solvent at least one compound corresponding to the following formula II:
(R₅ - E)ₘ- Cⱼ H_{2(j+1)-m-2d+i} Oₖ₊ᵢ
in which:
- R₅ is a long, saturated or unsaturated hydrocarbon chain having from 5 to 23 carbon atoms
- E is an 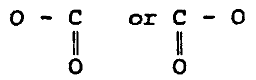 function
- m is an integer between 1 and 8
- j is an integer between 1 and 36
- k is an integer or zero
- i is an integer or zero
- d is equal to 0, 1 or 2.

17. Base according to Claim 16, characterized in that i is equal to 0.

18. Base according to Claim 17, characterized in that, if m is equal to 1, j is between 1 and 18, and preferably between 1 and 4, and k and d are equal to 0.

19. Base according to Claim 18, characterized in that, if j is equal to 1, R₅ is a hydrocarbon chain having from 7 to 17 carbon atoms.

20. Base according to Claim 17, characterized in that, if m is equal to 2, k and d are equal to 0 and j is between 2 and 12.

21. Base according to Claim 17, characterized in that, if m is equal to 2, k is equal to 1, j is between 4 and 12, d is equal to 0 and R₅ is a hydrocarbon chain having 7 or 9 carbon atoms.

22. Base according to Claim 17, characterized in that m is equal to 3, j is equal to 3 or 4, k and d are equal to 0 and R₅ is a hydrocarbon chain having 7 or 9 carbon atoms.

23. Base according to Claim 17, characterized in that, if m is equal to 3, j is equal to 6 and k and d are equal to 0.

24. Base according to Claim 17, characterized in that, if m is equal to 4, j is equal to 5 and k and d are equal to 0.

25. Base according to any one of Claims 16 to 24, characterized in that it comprises 60 to 99% by weight of at least one surfactant according to Claim 12 or 13 and 40 to 1% by weight of at least one compound corresponding to the formula (II).

26. Softening composition, characterized in that it comprises a base according to any one of Claims 14 to 25 and, optionally, additives.

27. Composition according to Claim 26, characterized in that it comprises at least one surfactant compound according to any one of Claims 2 to 13, the weight concentration of said surfactant being between 3 and 30% of the weight of said composition.

28. Composition according to claim 26, characterized in that it comprises at least one surfactant compound according to Claim 3, the weight concentration of said surfactant representing at least 60% by weight of said composition.

29. Composition according to Claim 28, characterized in that the remainder of said composition comprises by-products capable of being obtained by the condensation reaction between a glycerol triester and a tertiary amine, and in particular monoglycerides, diglycerides, glycerol.

30. Composition according to Claim 28, characterized in that the remainder of said composition comprises by-products capable of being obtained by the quaternization reaction of the condensation product, including said product unquaternized.

31. Process for directly preparing a mixture of surfactants according to Claim 12, according to which fatty acids corresponding, respectively, to the formulae R₁COOH, R₂COOH, R₃COOH in which R₁, R₂, R₃ are, respectively, a long, linear or branched alkyl or alkenyl hydrocarbon chain having from 5 to 23 carbon atoms, or their derivatives comprising their esters, anhydrides and acid chlorides, are reacted with at least one tertiary amine corresponding to the following formula (III): 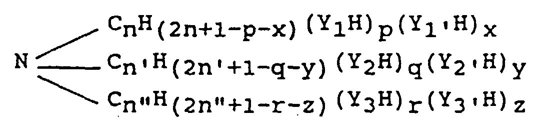 in which:
- n, n', n" are non-zero integers
- p, q, r, x, y, z are integers or zero
- expressed with reference to one mol, the mean of the sum of n, n' and n" is equal to at least 4
- Y₁, Y_{1'}, Y₂, Y_{2'}, Y₃, Y_{3'} are functional groups
and the condensation product thereby obtained is then reacted with an alkylating agent, in the presence or absence of a solvent
characterized in that the fatty acids are in free form and their molar concentration is between 1.85 and 1.40 times the overall molar concentration of tertiary amines each corresponding to the formula (III) in which, in addition:
- the sum of n, n' and n" is between 4 and 12,
- the sum of p, q, r, x, y and z is statistically on average between 1 and 4,
- Y₁, Y_{1'}, Y₂, Y_{2'}, Y₃, Y_{3'} are each chosen from oxy, N and NH groups.

32. Process for directly preparing a softening composition according to Claim 28, according to which fatty acids corresponding, respectively, to the formulae R₁COOH, R₂COOH, R₃COOH in which R₁, R₂, R₃ are, respectively, a long alkyl or alkenyl hydrocarbon chain having from 5 to 23 carbon atoms, said fatty acids being in the form of glyceride triesters, are reacted with at least one tertiary amine corresponding to the following formula (III) 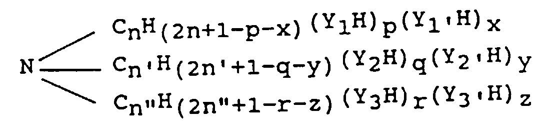 in which:
- n, n', n" are non-zero integers
- p, q, r, x, y, z are integers or zero
- expressed with reference to one mol, the mean of the sum of n, n' and n" is equal to at least 4
- Y₁, Y_{1'}, Y₂, Y_{2'}, Y₃, Y_{3'} are functional groups
and the condensation product thereby obtained is then reacted with an alkylating agent, in the presence or absence of a solvent
characterized in that
- expressed with reference to one mol, the mean of the sum of n, n' and n" is statistically between 4 and 12,
- expressed with reference to one mol, the mean of the sum of p, q, r, x, y and z is statistically between 1 and 4,
- Y₁, Y_{1'}, Y₂, Y_{2'}, Y₃, Y_{3'} are each chosen from oxy, N and NH groups,
and in that the tertiary amine is reacted partially, according to a transesterification and/or transesterification/amidation, with the glycerol triester.

33. Process according to Claim 32, characterized in that the mole ratio of the glycerol triester to the tertiary amine is between 1:3 and 1.

34. Process according to any one of Claims 31 to 33, characterized in that the alkylating agent is chosen from alkyl halides, sulphates, phosphates and carbonates.

35. Process according to Claim 32, characterized in that the condensation product is also reacted partially with the alkylating agent.

36. Process according to Claim 32, characterized in that the tertiary amine is monofunctional, that is to say contains a single hydroxyl or primary or secondary amine function, and is, in particular, chosen from the group comprising:
N,N-dimethylaminopropylamine
N,N-diethylaminopropylamine
N,N-dimethylaminoethanolamine
N,N-diethylaminoethanolamine
N,N-dimethylaminoisopropanolamine
N,N-diethylaminoisopropanolamine.

37. Process according to Claim 36, characterized in that at least one polyfunctional tertiary amine, that is to say containing several hydroxyl or primary or secondary amine functions, is combined with the monofunctional tertiary amine in a polyfunctional tertiary amine/monofunctional tertiary amine mole ratio of between 2 and 200.

38. Process according to Claim 37, characterized in that the polyfunctional tertiary amine is chosen from N,N-diethylamino-2,3-propanediol, N,N-dimethylamino-2,3-propanediol, methyldiethanolamine, ethyldiethanolamine and triethanolamine.
